# EUROPEAN PATENT APPLICATION

(11) **EP 4 311 827 A1**
(43) Date of publication of application: **31.01.2024**
(21) Application number: 22186662.7
(22) Date of filing: 25.07.2022
(51) Int. Cl.: C07C 67/54, C07C 67/08, C07C 69/24, B01D 3/14

(54) **METHOD AND PLANT FOR PURIFYING A FATTY ACID ISOPROPYL ESTER AND FOR PRODUCING PURE FATTY ACID ISOPROPYL ESTER**

(71) Applicant: Sulzer Management AG, 8401 Winterthur (CH)
(72) Inventor: The designation of the inventor has not yet been filed
(74) Representative: Henkel & Partner mbB

(57) **Abstract**

A method for purifying a fatty acid isopropyl ester comprising the following steps:
a) providing a crude fatty acid isopropyl ester composition containing a fatty acid isopropyl ester to be purified, at least one fatty acid and optionally at least one fatty acid ester being different from the fatty acid isopropyl ester to be purified, and
b) subjecting the crude fatty acid isopropyl ester composition provided in step a) to a distillation in a divided-wall column so as to produce an overhead stream, a bottom stream and a side stream, wherein the side stream is the purified fatty acid isopropyl ester composition.

## Description

The present invention relates to a method for purifying a fatty acid isopropyl ester. Moreover, the present invention relates to a method for preparing a pure fatty acid isopropyl ester and to a plant for preparing a pure fatty acid isopropyl ester.

While most of the fatty acid esters of methanol and acetic acid have a characteristic odor and taste and have thus only a limited use, such as for solvents or dispersing agents for medicaments and the like, fatty acid isopropyl esters are widely used in industry. For instance, fatty acid isopropyl esters are used as raw materials for various products, such as cosmetics, foods and pharmaceuticals, as ingredient of fragrances, as moisturizing agent, as emulsifier, as plasticizer, as thickener and others. More specifically, isopropyl laurate is a widely used ingredient of fragrances and cosmetics, such as lotions, soaps and shampoos. Furthermore, isopropyl myristate is a polar emollient and frequently used as disinfecting ingredient of mouthwashes, whereas isopropyl palmitate is used as emollient, moisturizer, thickener and antistatic agent. Typically, fatty acid isopropyl esters are produced by a catalyst assisted condensation reaction between a fatty acid, such as lauric acid, and isopropanol to the corresponding fatty acid isopropyl ester, such as isopropyl laurate, and water. In order to increase the yield of this reaction, at least some of the resulting water may be removed during the reaction together with some of the isopropanol from the mixture, for instance by distillation or stripping. The resulting reaction mixture includes the target fatty acid isopropyl ester, unreacted fatty acid, residual water, isopropanol, other impurities and byproducts. Most of the isopropanol and of the water is removed from the reaction mixture by separation, such as by a vaporization step, whereas the remaining crude mixture including the target fatty acid isopropyl ester, unreacted fatty acid, residual water, residual isopropanol, other impurities and byproducts is further purified. This further purification is typically done by two subsequent distillation steps. While in the first distillation step being performed in a first distillation column the low-boiling ingredients of the mixture, i.e. the ingredients having a lower boiling point than the target fatty acid isopropyl ester to be purified, are removed as overhead stream, the bottom product is fed into a second distillation column, in which the high-boiling ingredients of the mixture, i.e. the ingredients having a higher boiling point than the target fatty acid isopropyl ester to be purified, are removed as bottom stream. The purified target fatty acid isopropyl ester is obtained during the second distillation step as overhead or side fraction, which may then be further purified, for example by a stripping step with nitrogen.

While such an obtained purified fatty acid isopropyl ester composition has a high content of fatty acid isopropyl ester, such as more than 99% by weight, some critical impurities are still contained in a too high amount. These critical impurities are primarily unreacted fatty acids and not only the fatty acid corresponding to that fatty acid being contained as component in the purified fatty acid isopropyl ester, but also similar fatty acids. Similar fatty acid means a fatty acid differing from the fatty acid being contained as component in the target fatty acid isopropyl ester only in that it contains one to four less methylene groups or one to four more methylene groups. Such similar fatty acids are also designated as neighboring fatty acids. These similar fatty acids derive typically from the starting fatty acid composition, because purified fatty acid is never 100% pure, but still contains a certain amount of similar fatty acids. Thus, for instance palmitic acid typically contains small amounts of myristic acid, whereas myristic acid typically contains small amounts of lauric acid. A portion of these included similar fatty acids will be esterified during the condensation reaction to their corresponding fatty acid isopropyl esters, while the rest of these similar fatty acids remains unesterified in the reaction mixture. Other impurities are non-even fatty acids, such as undecylic acid, tridecylic acid, pentacylic acid and the like, palmitoleic acid and leaches from the acidic reaction catalyst. Unreacted fatty acids remaining in the purified fatty acid isopropyl ester lead to a comparable high acid value of the fatty acid isopropyl ester composition, such as of above 0.5 mg KOH/g of sample. However, for applications, such as the use in personal care products, cosmetics and pharma products, such as soaps, parfums, medicaments and the like, an extremely low acid value is an important purity criterion in addition to other purity criteria, such as absolute purity, water content, alcohol content and odor. However, a further reduction of the acid value, i.e. of the content of free fatty acids, and corresponding, similar free fatty acids, from a purified fatty acid isopropyl ester is not possible with the known two-distillation step purification methods. Other drawbacks of the known two-distillation step purification methods are their high operational costs (OPEX) and the required comparably high capital costs for the plant (CAPEX).

In view of this it is an object of the present invention to provide a method for the purification of a crude fatty acid isopropyl ester composition, which overcomes the aforementioned drawbacks, i.e. to provide a method for the purification of a crude fatty acid isopropyl ester composition, which results in a purified fatty acid isopropyl ester composition having an improved purity and in particular a reduced acid value, wherein the method is furthermore continuous, has a reduced energy consumption and requires a plant with comparable low investment costs.

In accordance with a first aspect of the present invention, this object is satisfied by providing a method for purifying a fatty acid isopropyl ester comprising the following steps:
a) providing a crude fatty acid isopropyl ester composition containing a fatty acid isopropyl ester to be purified, at least one fatty acid and optionally at least one fatty acid ester being different from the fatty acid isopropyl ester to be purified, and
b) subjecting the crude fatty acid isopropyl ester composition provided in step a) to a distillation in a divided-wall column so as to produce an overhead stream, a bottom stream and a side stream, wherein the side stream is the purified fatty acid isopropyl ester composition.

This solution bases on the finding that by using a distillation step in a divided-wall (distillation) column not only the operational costs and the required comparably high capital costs for the plant may be reduced, because not two, but only one distillation column is required and has to be operated, but that surprisingly and unexpectedly in particular also the purity of the resulting purified fatty acid isopropyl ester composition is significantly improved in comparison to a corresponding method comprising two subsequent traditional distillations in non-divided-wall columns, in particular by significantly reducing the acid value thereof. More specifically, the content of free fatty acids in the purified fatty acid isopropyl ester composition obtained with the process in accordance with the present invention may be reduced to about one third compared with the content of free fatty acids in the purified fatty acid isopropyl ester composition obtained with the aforementioned prior art approach basing on two subsequent distillation steps being not performed in divided-wall columns. It was found by the inventors of the present invention that the behavior of a fatty acid isopropyl ester and its corresponding fatty acid and similar fatty acids, such a neighboring fatty acids, is non-ideal. For instance, isopropyl palmitate and myristic acid form an azeotrope, whereas isopropyl palmitate and palmitic acid show a non-ideal vapor-/liquid equilibrium. Therefore, a further reduction of the content of free fatty acids and in particular of very similar free fatty acids from a purified fatty acid isopropyl ester is, if at all, only possible with a significant capacity reduction with the known purification methods basing on two subsequent distillation steps being not performed in divided-wall columns. However, the use of a divided-wall column surprisingly allows such a further reduction of the content of free fatty acids, presumably due to an increased overall reflux ratio of a divided-wall column in comparison to a traditional distillation column, due to the ratio of the reflux rate divided by the side stream rate and due to the fact that separation limits due to non-idealities of the vapor-/liquid equilibriums between an isopropyl ester and its corresponding or a similar free fatty acid are overcome.

The present invention is not particularly limited concerning the kind of fatty acid component of the fatty acid isopropyl ester, which is contained in the crude fatty acid isopropyl ester composition and which has to be purified. Preferably, the fatty acid component of the fatty acid isopropyl ester, which is contained in the crude fatty acid isopropyl ester composition and which has to be purified, is a saturated fatty acid meaning a fatty acid with a saturated aliphatic chain. More preferably, the fatty acid component of the fatty acid isopropyl ester, which is contained in the crude fatty acid isopropyl ester composition and which has to be purified, is an unbranched saturated fatty acid, yet more preferably an even-numbered unbranched saturated fatty acid and even more preferably an even-numbered C₆₋₂₂-unbranched saturated fatty acid. The fatty acid component of the fatty acid isopropyl ester, which is contained in the crude fatty acid isopropyl ester composition and which has to be purified, may not be a natural occurring fatty acid. However, it is preferred that it is a natural occurring fatty acid. Most preferably, the fatty acid component of the fatty acid isopropyl ester, which is contained in the crude fatty acid isopropyl ester composition and which has to be purified, is lauric acid, myristic acid, palmitic acid, stearic acid or an arbitrary mixture of two or more of the aforementioned fatty acids.

Thus, it is particularly preferred that the crude fatty acid isopropyl ester composition provided in step a) contains as fatty acid isopropyl ester to be purified isopropyllaurate, isopropylmyristate, isopropylpalmitate, isopropylstearate or an arbitrary mixture of two or more of the aforementioned fatty acid isopropyl esters.

Preferably, the crude fatty acid isopropyl ester composition provided in step a) is the reaction mixture obtained by a catalyst assisted condensation reaction between a fatty acid, preferably one of the aforementioned fatty acids, and isopropanol or a pre-purified reaction mixture obtained by a catalyst assisted condensation reaction between a fatty acid and by subsequently removing a portion of isopropyl alcohol and water from the reaction mixture. Such a crude fatty acid isopropyl ester composition contains the target fatty acid isopropyl ester to be purified, more or less isopropanol, more or less water, small amounts of other fatty acid isopropyl esters and small amounts of remaining fatty acids, in particular of the fatty acid corresponding to the fatty acid component of the target fatty acid isopropyl ester and of fatty acids being similar thereto.

For example, the crude fatty acid isopropyl ester composition provided in step a) contains 20 to 80% by weight and more preferably 30 to 70% by weight of the fatty acid isopropyl ester to be purified, 30 to 80 % by weight and more preferably 30 to 70% by weight of one or more fatty acids, 0.2 to 2.0 % by weight and more preferably 0.1 to 1.0% by weight of one or more fatty acid isopropyl esters being different from the fatty acid isopropyl ester to be purified, 0.001 to 1.0% by weight and more preferably 0.01 to 0.2% by weight of isopropanol and up to 0.5% by weight and more preferably 0.01 to 0.2% by weight of water, based on the total weight of the crude fatty acid isopropyl ester composition.

In accordance with the present invention, the crude fatty acid isopropyl ester composition provided in step a) is subjected in step b) to a distillation in a divided-wall column. The present invention is fundamentally not particularly limited concerning the kind of divided-wall column. Thus, in principle any divided-wall column may be used, i.e. any distillation column, which comprises at least one wall dividing at least an axial section of the interior of the distillation column in two different sub-sections.

In a further development of the idea of the present invention, it is suggested that the divided-wall column used in step b) comprises one dividing wall, which extends at least essentially vertically downwards. At least essentially vertically downwards means in this connection that the angle between the dividing wall and the length axis of the divided-wall column is at most 20°, preferably at most 10°, more preferably at most 5° and most preferably 0°.

The divided-wall column used in step b) may be a top divided-wall column, a middle divided-wall column or a bottom divided-wall column. In a top divided-wall column, the dividing wall is comprised in the top section of the distillation column, i.e. the dividing wall extends from the top of the distillation column (with the dividing wall being attached to the top of the distillation column) downwards in the direction to the bottom of the distillation column by a certain extent, such as - seen from the top to the bottom of the distillation column - up to 90%, up to 80%, up to 70%, up to 60%, up to 50% or up to 40% of the height of the distillation column. Thus, the bottom section of a top divided-wall column remains undivided. Consequently, the dividing wall ends, seen from the top to the bottom of the distillation column - at least 10% or at least 20% or at least 30% or at least 40% or at least 50% or at least 60% of the column height above the bottom of the distillation column.

Analogous thereto, the dividing wall of a bottom divided-wall column is comprised in the bottom section of the distillation column, i.e. the dividing wall extends from the bottom of the distillation column (with the dividing wall being attached to the bottom of the distillation column) upwards in the direction to the top of the distillation column by a certain extent, such as - seen from the bottom to the top of the distillation column - up to 90%, up to 80%, up to 70%, up to 60%, up to 50% or up to 40% of the height of the distillation column. Consequently, the dividing wall ends, seen from the bottom to the top of the distillation column - at least 10% or at least 20% or at least 30% or at least 40% or at least 50% or at least 60% of the column height below the top of the distillation column. Thus, the top section of the distillation column remains undivided.

Analogous thereto, the dividing wall of a middle divided-wall column is comprised in the central section of the distillation column, i.e. the dividing wall does neither touch the top nor the bottom of the divided-wall column, but extends so that the top section as well as the bottom section of the distillation column remain undivided. It is preferred that the dividing wall of the middle divided-wall column extends, seen along the length axis of the middle divided-wall column, from at least 10 to at most 90%, preferably from at least 20 to at most 80%, and more preferably from at least 25 to at most 25% of the height of the divided-wall column. In other words, the dividing wall of the middle divided-wall column has a distance to the top as well as to the bottom of the middle divided-wall column, independently from each other, of at least 10% or of at least 20% or of at least 25% of the height of the divided-wall column. For instance, the dividing wall of the middle divided-wall column extends, seen from the top to the bottom along the length axis of the middle divided-wall column, from 10 to 70% or from 20 to 75% or from 25 to 75% of the height of the divided-wall column.

Independently from whether a top divided-wall column, a middle divided-wall column or a bottom divided-wall column is used, it is preferred that the dividing wall divides the axial section of the distillation column, seen in cross-section of the distillation column, in about two equally sized sub-sections or halves, respectively. That means that the dividing wall is arranged, based on the cross-section of the divided-wall column, so as to divide the cross-section of the divided-wall column into two sub-sections, wherein each of the two sub-sections covers at least 40%, more preferably at least 45% and most preferably 50% of the total area of the cross-section of the divided-wall column, with the sum of both sub-section coverages being of course 100%. For instance, one sub-section covers 43% and the other 57% of the total area of the cross-section or both sub-sections cover each exactly 50% of the total area of the cross-section.

In accordance with a particular preferred embodiment of the present invention the divided-wall column used in step b) is a middle divided-wall column comprising:
- an undivided top section for concentrating low-boiling components comprising an outlet for the overhead stream of the low-boiling components, wherein the low-boiling components have a lower boiling point than the fatty acid isopropyl ester to be purified,
- an undivided bottom section for concentrating high-boiling components comprising an outlet for the bottom stream of the high-boiling components, wherein the high-boiling components have a higher boiling point than the fatty acid isopropyl ester to be purified, and
- a central section arranged between the top section and the bottom section having at least one dividing wall for dividing the central section into at least one feed sub-section and at least one product withdrawal sub-section, the feed sub-section having an inlet for the feed of the crude fatty acid isopropyl ester composition and the product withdrawal sub-section having a side draw outlet for the purified fatty acid isopropyl ester composition.

Section means in this connection an axial extending section of the interior of the distillation column, wherein the sum of all three sections, i.e. the top section, the bottom section and the central section form the total interior of the distillation column. Undivided section means that the section does not contain any wall, let alone any wall dividing the section into two or more sub-sections.

In accordance with a further particularly preferred embodiment of the present invention, the side draw outlet for the purified fatty acid isopropyl ester composition is arranged in the central section of the middle divided-wall column above the inlet for the feed of the crude fatty acid isopropyl ester composition. This means that the side draw outlet for the purified fatty acid isopropyl ester composition is arranged in the product withdrawal sub-section, seen in the length direction of the distillation column from the bottom to the top, vertically above the position at which the inlet for the feed of the crude fatty acid isopropyl ester composition is arranged in the feed sub-section of the divided-wall column. It has been found by the inventors of the present invention that thereby a particular efficient separation of free fatty acids and in particular of fatty acid corresponding to the fatty acid component of the target fatty acid isopropyl ester and fatty acids being similar thereto is achieved.

In a further development of the idea of the present invention, it is proposed that each of the feed sub-section and of the product withdrawal sub-section of the central section of the divided-wall column comprises at least two packing beds, wherein adjacent packing beds are arranged on top of each other. Such packing beds are usually used in distillation columns in order to increase the contact area between the liquid phase descending the distillation column and the gas phase ascending the distillation column during its operation. Preferably, a distributor is arranged on top of each packing bed in order to evenly distribute the liquid phase over the cross-section of the bed, while leaving enough space for the gas phase to ascend through it. Moreover, a grid-like holding device and a collector are preferably arranged below each packing bed, wherein the grid-like structure keeps the packing bed at its position and the collector collects the liquid phase trickling down from the packing bed, while sufficient open space is left in the collector for the gas phase to ascend.

Each of the packing beds may comprise one or more structured packing elements or a random packing. A structured packing bed comprises one or more layers of structured packing elements being stacked vertically above each other, wherein each layer is composed of one or more structured packing elements. The higher the diameter of the structured packing bed, the higher the number of structured packing elements per layer. The structured packing elements comprise a plurality of different sheets, each of which providing surface area for the liquid phase that trickles down along the surface of the sheet and spreads. In addition, between the different sheets of the structured packing elements open spaces are provided, which are filled with the gas phase and provide a path for the gas phase to ascend, while it is driven by a pressure gradient. By allowing the liquid phase to spread on the surface of the structured packing elements, an interface is created between the liquid phase and the ascending gas phase so that an efficient heat and mass transfer between the two phases is established at the interface.

Preferably, each of the structured packing elements is a cross-channel corrugated sheet packing, which is assembled from a plurality of corrugated sheets, which are arranged parallel and in touching contact with each other. Typically, the corrugated metal sheets are fixed with each other by means of several rods penetrating the corrugated sheets perpendicular to the longitudinal section of the corrugated sheets, wherein the rods are fixed with the first and last corrugated sheet by means of a washer and a nut or by bending the rod. Each corrugated sheet comprises a plurality of alternately oriented peaks and valleys, wherein adjacent corrugated sheets are oriented such that the corrugations of the adjacent corrugated sheets intersect in crisscross fashion, with the corrugations of the corrugated sheets extending obliquely relative to the vertical or longitudinal direction, thus forming inclined channels which continuously cross one another. These inclined channels positively influence the flows of the gas phase and of the liquid phase within the packing and facilitate the mass and/or heat transfer between the phases.

Good results are in particular obtained, when each of the feed sub-section and of the product withdrawal sub-section of the central section of the divided-wall column comprises at least three packing beds, more preferably at least four packing beds and most preferably four packing beds, wherein adjacent packing beds are arranged on top of each other, and wherein each of the packing beds preferably comprises one or more structured packing elements or a random packing. All packing beds completely cover the surface area of the corresponding central section, i.e. the sum of the feed sub-section and of the product withdrawal sub-section. Each of the structured packing elements may having a specific surface area in the range of 125 to 900 m²/m³, preferably of 200 to 750 m²/m³ and more preferably of 250 to 500 m²/m³. All of the packing beds may have the same height or a different height. Moreover, the packing beds of the feed-sub-section and the packing beds of the withdrawal-sub-section may be arranged parallel to each other, i.e. the lower or upper ends of the packing beds of the feed-sub-section are, seen in the length direction of the distillation column, at the same height than the respective ends of the packing beds of the withdrawal-sub-section.

In a further development of the idea of the present invention, it is suggested that the inlet for the feed of the crude fatty acid isopropyl ester composition is located between two adjacent packing beds of the feed sub-section and the outlet for the purified fatty acid isopropyl ester composition is located between two adjacent packing beds of the product withdrawal sub-section, wherein preferably the side draw outlet for the purified fatty acid isopropyl ester composition is arranged in the withdrawal-sub-section of the central section of the middle divided-wall column above the inlet for the feed of the crude fatty acid isopropyl ester composition.

In a preferred specific embodiment of the present invention, each of the feed sub-section and of the product withdrawal sub-section of the central section of the middle divided-wall column comprises four packing beds, wherein, seen from the bottom to the top of the divided-wall column, the inlet for the feed of the crude fatty acid isopropyl ester composition is located between the first and second of adjacent packing beds of the feed sub-section, and wherein the outlet for the purified fatty acid isopropyl ester composition is located between the third and fourth of adjacent packing beds of the product withdrawal sub-section of the divided-wall column. Again, all of the packing beds may have the same height or a different height. Moreover, the packing beds of the feed-sub-section and the packing beds of the withdrawal-sub-section may be arranged parallel to each other, i.e. the lower or upper ends of the packing beds of the feed-sub-section are, seen in the length direction of the distillation column, at the same height than the respective ends of the packing beds of the withdrawal-sub-section.

In an alternative preferred specific embodiment of the present invention, each of the feed sub-section and of the product withdrawal sub-section of the central section of the divided-wall column comprises four packing beds, wherein, seen from the bottom to the top of the divided-wall column, the inlet for the feed of the crude fatty acid isopropyl ester composition is located between the second and third of adjacent packing beds of the feed sub-section, and wherein the outlet for the purified fatty acid isopropyl ester composition is located between the second and third of adjacent packing beds of the product withdrawal sub-section, wherein the upper end of the second packing bed of the product withdrawal sub-section is above the lower end of the third packing bed of the feed sub-section of the divided-wall column. In other words, also in this embodiment the outlet for the purified fatty acid isopropyl ester composition is located above the inlet for the feed of the crude fatty acid isopropyl ester composition.

The top section and the bottom section of the middle divided-wall column, which are located above and below the central section, do not need to include a packing bed. However, preferably the top section of the middle divided-wall column comprises at least one and preferably one packing bed and/or the bottom section of the middle divided-wall column comprises at least one and preferably one packing bed. More preferably, the top section of the middle divided-wall column comprises one packing bed and the bottom section of the middle divided-wall column comprises one packing bed. Again, each of the packing beds may comprise one or more structured packing elements or a random packing.

Good results in terms of separation efficiency in particular between the target fatty acid isopropyl ester to be purified and the fatty acid corresponding to the fatty acid component of the target fatty acid isopropyl ester and fatty acids being similar thereto, are in particular obtained, when the divided-wall column has during its operation in each of the feed sub-section and of the product withdrawal sub-section at least 40, preferably at least 45, more preferably at least 50 and more preferably at least 60 theoretical stages. The feed sub-section may have the same number of theoretical stages or a different number of theoretical stages as the product withdrawal sub-section. For instance, the feed sub-section may have 50 theoretical stages and the product withdrawal sub-section 42 theoretical stages, or feed sub-section as well as the product withdrawal sub-section have each 50 theoretical stages. Preferably, the feed sub-section has the same number of theoretical stages as the product withdrawal sub-section.

Preferably, the pressure and temperature at the bottom of the divided wall column are during the operation of the divided-wall column in the ranges of 5 to 100 mbar and 150 to 300°C, more preferably of 10 to 60 mbar and of 180 to 280°C and yet more preferably of 30 to 50 mbar and of 220 to 240°C. Furthermore, it is preferred that the pressure and temperature at the head of the divided wall column are during the operation of the divided-wall column in the ranges of 1 to 100 mbar and 100 to 280°C, more preferably of 12 to 30 mbar and of 150 to 220°C and yet more preferably of 10 to 20 mbar and of 180 to 200°C, with the temperature at the head of the divided wall column being lower than the temperature at the bottom of the divided wall column.

In a further development of the idea of the present invention, it is proposed that the divided-wall column used in step b) has an overhead condenser being in fluid communication with the head and a reboiler being in fluid communication with the bottom of the middle dividing-wall distillation column. More specifically, it is preferred that the divided-wall column used in step b) has an overhead condenser having an inlet being connected via a line with the overhead or another part of the top section of the divided-wall column and having an outlet for condensed liquid being connected with a line, which splits into a liquid removal line and into a recycle line for recycling a portion of the liquid having been condensed in the condenser into the top section of the dividing-wall distillation column. Likewise thereto, it is preferred that the divided-wall column used in step b) has a reboiler having an inlet being connected via a line with the bottom or with another part of the bottom section of the divided-wall column and having an outlet for a gas stream being connected with a line, which splits into a gas removal line and into a recycle line for recycling a portion of the gas having been generated in the reboiler into the bottom section of the dividing-wall distillation column.

According to a particular preferred embodiment of the present invention, the reflux ratio is 200 to 1,000, preferably 300 to 900 and more preferably 500 to 800. The reflux ratio is the ratio of the amount of liquid being condensed in the overhead condenser and being recycled into the divided-wall column divided by the amount of liquid being condensed in the overhead condenser and being withdrawn from the distillation column, for example via the liquid removal line.

Moreover, it is preferred that the ratio of the reflux rate divided by the side stream rate is 4 to 10, more preferably 5 to 8 and most preferably 6 to 7. The ratio of the reflux rate divided by the side stream rate is the amount of liquid being condensed in the overhead condenser and being recycled into the divided-wall column divided by the amount of the side stream removed from the divided-wall column.

In view of the separation efficiency of the divided-wall column used in step b), the method in accordance with the present invention does not need any further distillation step to be performed in addition to the distillation step being performed in the divided-wall column.

Optionally, the purified fatty acid isopropyl ester composition obtained in step b) of the method may be, but must not be further subjected in a step c) to a stripping step, such as to a stripping step with nitrogen. Thereby, potential remaining odor causing compounds may be completely removed.

As set out above, the method in accordance with the present invention is characterized by a particular good separation efficiency of fatty acid and in particular fatty acid corresponding to the fatty acid component of the target fatty acid isopropyl ester and fatty acids being similar thereto from the target fatty acid isopropyl ester. Accordingly, it is preferred that the purified fatty acid isopropyl ester composition obtained in step b) of the method in accordance with the present invention as side stream comprises at least 99.00% by weight, preferably at least 99,30%, more preferably at least 99,50%, even more preferably at least 99,60% and most preferably at least 99,70% of the target fatty acid isopropyl ester.

Likewise thereto, it is preferred that the purified fatty acid isopropyl ester composition obtained in step b) of the method in accordance with the present invention as side stream comprises at most 0.070% by weight, preferably at most 0.060% by weight, more preferably at most 0.050% by weight, even more preferably at most 0.040% by weight and most more preferably at most 0.030% by weight of impurities comprising free fatty acid and other fatty acid isopropyl esters than the target fatty acid isopropyl ester.

Thereby, a purified fatty acid isopropyl ester composition with an acid value of less than 1.0 mg KOH/g of sample, preferably of less than 0.5 mg KOH/g of sample, more preferably of less than 0.4 mg KOH/g of sample and most preferably of less than 0.3 mg KOH/g of sample is obtained. The acid value of a fatty acid isopropyl ester composition is determined in accordance with the present invention by titration or by gel chromatography. And more preferably by AOCS Te 1a-64.

Preferably, the overhead stream obtained in step b) comprises at most 80% by weight and more preferably at most 70% by weight of the target fatty acid isopropyl ester to be purified. In turn, the overhead stream obtained in step b) preferably comprises at least 20% by weight and more preferably at least 30% by weight of sum of fatty acids, of water, of isopropanol and of fatty acid isopropyl esters being different to the target fatty acid isopropyl ester to be purified.

Preferably, the bottom stream obtained in step b) comprises at most 30% by weight, more preferably at most 20% by weight, even more preferably at most 15% by weight and most preferably at most 10% by weight of the target fatty acid isopropyl ester to be purified. In turn, the bottom stream obtained in step b) preferably comprises at least 70% by weight, more preferably at least 80% by weight, even more preferably at least 85% by weight and most preferably at least 90% by weight of sum of fatty acids and of fatty acid isopropyl esters being different to the fatty acid isopropyl ester to be purified.

In accordance with a second aspect, the present invention is related to a method for preparing a pure fatty acid isopropyl ester comprising the following steps:
i) providing a starting mixture containing a fatty acid and isopropanol,
ii) reacting the starting mixture preferably in the presence of a catalyst so as to obtain a reaction mixture containing the fatty acid isopropyl ester, unreacted fatty acid, unreacted isopropanol and optionally water, by-products and other impurities,
iii) optionally, removing at least a part of the isopropyl from the reaction mixture by vaporization so as to obtain a modified reaction mixture, and
iv) performing a method for purifying the fatty acid isopropyl ester as described above, wherein the crude fatty acid isopropyl ester composition provided in step a) is the reaction mixture obtained in step ii) or the modified reaction mixture obtained in step iii).

All features described above with regard to the first aspect of the present invention, i.e. with regard to the method for purifying a fatty acid isopropyl ester, are also preferred for the second aspect of the present invention, i.e. with regard to step iv) of the method for preparing a pure fatty acid isopropyl ester.

In particular, the purified fatty acid isopropyl ester composition obtained in step b) of step iv) may be further subjected to a stripping step, such as a stripping step with nitrogen.

In accordance with a third aspect, the present invention is related to a plant for preparing a pure fatty acid isopropyl ester comprising:
i) at least one reactor comprising one or more inlets for fatty acid and isopropanol or a mixture of fatty acid and isopropanol, and an outlet for a reaction mixture containing the fatty acid isopropyl ester, unreacted fatty acid, unreacted isopropanol and optionally water, by-products and other impurities,
ii) optionally, a separation means for separating at least a part of isopropanol from the reaction mixture so as to obtain a modified reaction mixture, wherein the separation means comprises an inlet being connected with the outlet for the reaction mixture of the reactor, an outlet for isopropanol and an outlet for the modified reaction mixture, and
iii) a divided-wall column comprising an inlet being connected with the outlet for the reaction mixture of the reactor and/or with the outlet for the modified reaction mixture of the separation means.

For instance, the reactor a) is a vessel containing a catalyst.

In an alternative embodiment of the present invention, at least one reactor a) is a distillation column comprising as bottom outlet the outlet for the reaction mixture and an overhead outlet, wherein the overhead outlet is connected via a line with a water removal unit, wherein the water removal unit comprises an outlet for water and an outlet for isopropanol, wherein the outlet for isopropanol is connected via a recycle line with the inlet for isopropanol of the reactor a) or (indirectly) with the inlet for a mixture of fatty acid and isopropanol of the reactor a). In the latter alternative, the outlet for isopropanol is connected with a vessel for mixing isopropanol and fatty acid, wherein the vessel has an outlet line connecting the vessel and the inlet for the mixture of fatty acid and isopropanol. Thereby, isopropanol and the reaction product water may be continuously removed during the reaction from the mixture so as to shift the reaction equilibrium to the side of the products.

In still another embodiment of the present invention, the plant may comprise two reactors, wherein the first reactor functions as pre-reactor and is a vessel comprising one or more inlets for fatty acid and isopropanol or a mixture of fatty acid and isopropanol, and an outlet for a pre-reaction mixture containing the fatty acid isopropyl ester, unreacted fatty acid, unreacted isopropanol and optionally water, by-products and other impurities. The second reactor may be a distillation column comprising an inlet being connected with the outlet for the pre-reaction mixture of the first reactor, as bottom outlet the outlet for the (final) reaction mixture and an overhead outlet, wherein the overhead outlet is connected via a line with a water removal unit. The water removal unit comprises an outlet for water and an outlet for isopropanol, wherein the outlet for isopropanol is connected via a recycle line with the inlet for isopropanol of the first reactor or (indirectly) with the inlet for a mixture of fatty acid and isopropanol of the first reactor. In the latter alternative, the outlet for isopropanol is connected with a vessel for mixing isopropanol and fatty acid, wherein the vessel has an outlet line connecting the vessel and the inlet for the mixture of fatty acid and isopropanol. Thereby, isopropanol and the reaction product water may be continuously removed during the reaction from the mixture so as to shift the reaction equilibrium to the side of the products.

The water removal unit may be a vaporizer, a distillation column and is preferably a pervaporation unit.

In accordance with a further preferred embodiment of the present invention, the plant comprises the optional separation means b), wherein the outlet for isopropanol of the separation means b) is connected via a recycle line with the inlet for isopropanol of the reactor a) or with the inlet for a mixture of fatty acid and isopropanol of the reactor a), wherein the outlet for isopropanol is connected with a vessel for mixing isopropanol and fatty acid, with an outlet line connecting the vessel and the inlet for the mixture of fatty acid and isopropanol.

The separation means may be a distillation column and is preferably a vaporizer.

All features described above with regard to the first aspect of the present invention, i.e. with regard to the method for purifying a fatty acid isopropyl ester, are also preferred for the third aspect of the present invention. In particular, all features described above with regard to the divided-wall distillation column of the first aspect of the present invention are also preferred for the divided-wall distillation column iii) of the third aspect of the present invention.

Thus, the divided-wall column is preferably a middle divided-wall column comprising one dividing wall, which extends at least essentially vertically downwards, i.e. wherein the angle between the dividing wall and the length axis of the divided-wall column is at most 20°, preferably at most 10°, more preferably at most 5° and most preferably 0°.

Preferably, the dividing wall is comprised in the central section of the distillation column, i.e. the dividing wall does neither touch the top nor the bottom of the divided-wall column, but extends so that the top section as well as the bottom section of the distillation column remain undivided. It is preferred that the dividing wall of the middle divided-wall column extends, seen along the length axis of the middle divided-wall column, from at least 10 to at most 90%, preferably from at least 20 to at most 80%, and more preferably from at least 25 to at most 25% of the height of the divided-wall column.

In addition, it is preferred that the dividing wall divides the axial section of the distillation column, seen in cross-section of the distillation column, into two sub-sections, wherein each of the two sub-sections covers at least 40%, more preferably at least 45% and most preferably 50% of the total area of the cross-section of the divided-wall column, with the sum of both sub-section coverages being of course 100%.

In accordance with a particular preferred embodiment of the present invention the divided-wall column iii) is a middle divided-wall column comprising:
- an undivided top section for concentrating low-boiling components comprising an outlet for the overhead stream of the low-boiling components, wherein the low-boiling components have a lower boiling point than the fatty acid isopropyl ester to be purified,
- an undivided bottom section for concentrating high-boiling components comprising an outlet for the bottom stream of the high-boiling components, wherein the high-boiling components have a higher boiling point than the fatty acid isopropyl ester to be purified, and
- a central section arranged between the top section and the bottom section having at least one dividing wall for dividing the central section into at least one feed sub-section and at least one product withdrawal sub-section, the feed sub-section having an inlet for the feed of the crude fatty acid isopropyl ester composition and the product withdrawal sub-section having a side draw outlet for the purified fatty acid isopropyl ester composition.

In accordance with a further particularly preferred embodiment of the present invention, the side draw outlet for the purified fatty acid isopropyl ester composition is arranged in the product withdrawal sub-section of the central section of the middle divided-wall column above the inlet for the feed of the crude fatty acid isopropyl ester composition.

In a further development of the idea of the present invention, it is proposed that each of the feed sub-section and of the product withdrawal sub-section of the central section of the divided-wall column comprises at least two, preferably at least three, more preferably at least four and most preferably four packing beds being arranged on top of each other. Each of the packing beds preferably comprises one or more structured packing elements, which are more preferably cross-channel corrugated sheet packings.

In a particular preferred embodiment of the present invention, the inlet for the feed of the crude fatty acid isopropyl ester composition is located between two adjacent packing beds of the feed sub-section and the outlet for the purified fatty acid isopropyl ester composition is located between two adjacent packing beds of the product withdrawal sub-section, wherein the side draw outlet for the purified fatty acid isopropyl ester composition is arranged in the product withdrawal sub-section if the central section of the middle divided-wall column above the inlet for the feed of the crude fatty acid isopropyl ester composition.

In a preferred specific embodiment of the present invention, each of the feed sub-section and of the product withdrawal sub-section of the central section of the middle divided-wall column comprises four packing beds, wherein, seen from the bottom to the top of the divided-wall column, the inlet for the feed of the crude fatty acid isopropyl ester composition is located between the first and second of adjacent packing beds of the feed sub-section, and wherein the outlet for the purified fatty acid isopropyl ester composition is located between the third and fourth of adjacent packing beds of the product withdrawal sub-section of the divided-wall column. All of the packing beds may have the same height or a different height. Moreover, the packing beds of the feed-sub-section and the packing beds of the withdrawal-sub-section may be arranged parallel to each other, i.e. the lower or upper ends of the packing beds of the feed-sub-section are, seen in the length direction of the distillation column, at the same height than the respective ends of the packing beds of the withdrawal-sub-section.

In an alternative preferred specific embodiment of the present invention, each of the feed sub-section and of the product withdrawal sub-section of the central section of the divided-wall column comprises four packing beds, wherein, seen from the bottom to the top of the divided-wall column, the inlet for the feed of the crude fatty acid isopropyl ester composition is located between the second and third of adjacent packing beds of the feed sub-section, and wherein the outlet for the purified fatty acid isopropyl ester composition is located between the second and third of adjacent packing beds of the product withdrawal sub-section, wherein the upper end of the second packing bed of the product withdrawal sub-section is above the lower end of the third packing bed of the feed sub-section of the divided-wall column. In other words, the outlet for the purified fatty acid isopropyl ester composition is located above the inlet for the feed of the crude fatty acid isopropyl ester composition.

The top section and the bottom section of the middle divided-wall column, which are located above and below the central section, do not need to include an packing bed. However, preferably the top section of the middle divided-wall column comprises at least one and preferably one packing bed and/or the bottom section of the middle divided-wall column comprises at least one and preferably one packing bed. More preferably, the top section of the middle divided-wall column comprises one packing bed and the bottom section of the middle divided-wall column comprises one packing bed.

Subsequently, the present invention is described by means of illustrative, but not limiting figures, in which:
- Fig. 1: shows a schematic longitudinal section of a divided-wall column being suitable to be used in a method for purifying a fatty acid isopropyl ester in accordance with one embodiment of the present invention and
- Fig. 2: shows a schematic view of a plant for preparing a pure fatty acid isopropyl ester in accordance with another embodiment of the present invention.

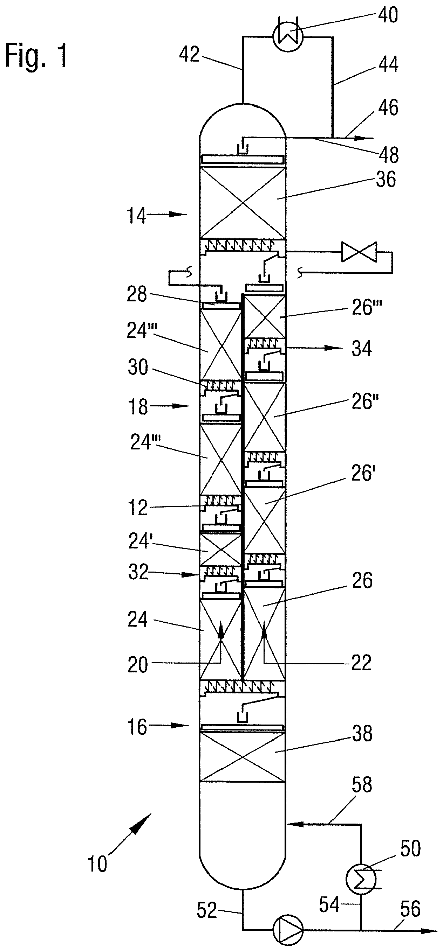

The divided-wall column 10 shown schematically in figure 1 is a middle divided-wall column 10, which comprises a dividing wall 12, which extends, seen over the height of the divided-wall column 10, vertically downwards. More specifically, the dividing wall 12 extends between the top and the bottom of the divided-wall column 14 vertically downwards from about 25% to about 75% of the height of the middle divided-wall column. Consequently, the dividing wall 12 comprises an undivided top section 14, an undivided bottom section 16 and a central section 18 being arranged between the top section 14 and the bottom section 16. The dividing wall 12 extends over the whole height of the central section 18, but not within the top section 14 and not within the bottom section 16. Furthermore, the dividing wall 12 divides the central section 18 of the divided-wall column 12 into a first, feed sub-section 20 and into a second, product withdrawal sub-section 22.

The feed sub-section 20 of the central section 18 of the divided-wall column 10 comprises four packing beds 24, 24', 24", 24", which are arranged on top of each other and each of which comprises several structured packing elements. Also the product withdrawal sub-section 22 of the central section 18 of the divided-wall column 10 comprises four packing beds 26, 26', 26", 26"', which are arranged on top of each other and each of which comprises several structured packing elements. The packing beds 24, 24', 24", 24", 26, 26', 26", 26'" have different heights and the packing beds 24, 24', 24", 24" of the feed sub-section 20 are not parallel to the packing beds 26, 26', 26", 26"' of the product withdrawal sub-section 22, but displaced to some extent. Above each of the packing beds 24, 24', 24", 24", 26, 26', 26", 26'" a distributor 28 is provided and below each of the packing beds 24, 24', 24", 24" a collector 30 is provided. The divided-wall column 10 further comprises an inlet being connected with a feed inlet line 32 and a side draw outlet being connected with a product withdrawal line 34. While the feed inlet line 32 is positioned at a height between the first packing bed 24 and the second packing bed 24' of the feed sub-section 20 of the central portion 18 of the divided-wall column 10, the product withdrawal line 34 is positioned at a height between the third packing bed 26" and the fourth packing bed 26'" of the product withdrawal sub-section 22 of the central portion 18 of the divided-wall column 10. Thus, the product withdrawal line 34 is positioned, seen from the bottom to the top of the divided-wall column 10, above the feed inlet line 32. The undivided top section 14 of the divided-wall column 10 comprises a packing bed 36 and the undivided bottom section 16 of the divided-wall column 10 also comprises a packing bed 38.

In addition, the divided-wall column 10 comprises an overhead condenser 40, which has an inlet being connected via a line 42 with the overhead of the top section 14 of the divided-wall column 10 and has an outlet being connected with a line 44 for condensed liquid, which splits into a liquid removal line 46 and into a recycle line 48 for recycling a portion of the liquid having been condensed in the condenser 40 into the top section 14 of the dividing-wall column 10. Moreover, the divided-wall column 10 comprises a reboiler 50 and a bottom outlet line 52, which splits off into a line 54 being connected with the inlet of the reboiler 50 and into a removal line 56. The reboiler 50 has further an outlet being connected with a line a recycle line 58 for recycling a portion of the gas having been generated in the reboiler 50 into the bottom section 16 of the dividing-wall column 10.

During the operation, a crude fatty acid isopropyl ester composition containing a fatty acid isopropyl ester to be purified, at least one fatty acid and optionally at least one fatty acid ester being different from the fatty acid isopropyl ester to be purified is fed via the feed inlet line 32 into the feed section 20 of the central section 18 of the dividing-wall column 10 and is distilled. The low-boiling ingredients of the crude fatty acid isopropyl ester composition, i.e. the ingredients having a lower boiling point than the target fatty acid isopropyl ester to be purified, are removed as overhead stream via line 42. The overhead stream is condensed in the condenser 40 and a part of the condensed liquid of the low-boiling ingredients is withdrawn from the dividing-wall column 10 via line 46, whereas the rest thereof is recycled into the top section 14 of the dividing-wall column 10 via the recycle line 48. The high-boiling ingredients of the crude fatty acid isopropyl ester composition, i.e. the ingredients having a higher boiling point than the target fatty acid isopropyl ester to be purified, are removed from the divided-wall column 10 as bottom stream via line 52. A part of the bottom stream is led via line 54 into the reboiler 50 and is evaporated therein and recycled via the recycle line 58, whereas the other part of the bottom stream of the high-boiling ingredients is withdrawn from the dividing-wall column 10 via line 56. The obtained purified fatty acid isopropyl ester composition is withdrawn from the dividing-wall column 10 via the product withdrawal line 34.

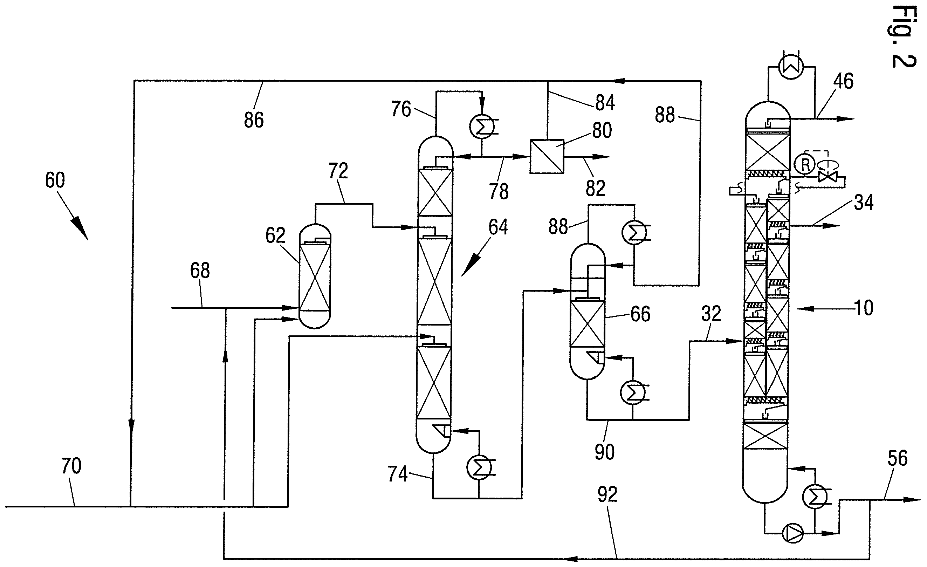

The plant 60 for preparing a pure fatty acid isopropyl ester shown in figure 2 comprises a first reactor 62 or pre-reactor 62, respectively, a second reactor 64 and a separation means 66. The pre-reactor 62 is a vessel comprising an inlet line 68 for fatty acid, an inlet line 70 for isopropanol and an outlet line 72 for a pre-reaction mixture containing the fatty acid isopropyl ester, unreacted fatty acid, unreacted isopropanol and optionally water, by-products and other impurities. The second reactor 64 is a distillation column comprising an inlet being connected with the outlet line 72 for the pre-reaction mixture of the pre-reactor 62, as bottom outlet an outlet line 74 for the (final) reaction mixture and an overhead outlet line 76, wherein the overhead outlet line 76 is connected via a line 78 with a water removal unit 80. The water removal unit 80, which is in fact a pervaporation unit, comprises an outlet line 82 for water and an outlet line 84 for isopropanol, wherein the outlet 84 for isopropanol is connected via a recycle line 86 with the inlet line 70 for isopropanol of the pre-reactor 62. The separation means 66 is a distillation column having an overhead line 88 for isopropanol leading into the recycle line 86 and a bottom line 90 for removing the modified reaction mixture and feeding it via the feed inlet line 32 into the divided-wall column 10, which is embodied as described above with regard to figure 1. From the bottom removal line 56 of the divided-wall column 10 a recycle line 92 leads back into the inlet line 68 for fatty acid.

During the operation of the plant 60, fatty acid and isopropanol are fed via the inlet lines 68 and 70 into the pre-reactor 62, in which these are reacted to fatty acid isopropyl ester. The pre-reaction mixture is withdrawn from the pre-reactor 62 and fed into the second reactor 64 via line 72, in which the reaction of fatty acid and isopropanol is completed. A mixture of isopropanol and water is continuously removed from the second reactor 64 through the overhead outlet line 76 and led into the water removal unit 80, in which isopropanol is separated from water. While the water is withdrawn via the outlet line 82, the isopropanol is recycled into the pre-reactor 62 via the recycle line 86 and via the isopropanol inlet line 70. The reaction mixture is withdrawn from the second reactor 64 via the outlet line 74 and fed into the separation means 66. In the separation means 66 a major part of the remaining isopropanol is removed from the reaction mixture, which is withdrawn from the separation means 66 via the overhead outlet line 88 and fed back into the pre-reactor via the recycle line 86 and via the isopropanol inlet line 70. The so obtained modified reaction mixture is removed from the separation means 66 via the bottom line 90, fed into the divided-wall column 10 via the feed inlet line and purified in the divided-wall column 10 as described above with regard to figure 1.

### Reference numerals

- 10: Divided-wall column
- 12: Dividing wall of divided-wall column
- 14: Undivided top section
- 16: Undivided bottom section
- 18: Central section
- 20: Feed sub-section of the central section
- 22: Product withdrawal sub-section of the central section
- 24, 24', 24", 24‴: Packing bed of the feed sub-section
- 26, 26', 26", 26"': Packing bed of the product withdrawal sub-section
- 28: Distributor
- 30: Collector
- 32: Feed inlet line
- 34: Product withdrawal line
- 36: Packing bed of the undivided top section
- 38: Packing bed of the undivided bottom section
- 40: Overhead condenser
- 42: Line to inlet of the condenser
- 44: Line from the outlet of the condenser
- 46: Liquid removal line
- 48: Recycle line for liquid
- 50: Reboiler
- 52: Bottom outlet line
- 54: Line to inlet of the reboiler
- 56: Removal line
- 58: Recycle line for gas
- 60: Plant for preparing a pure fatty acid isopropyl ester
- 62: First reactor / pre-reactor
- 64: Second reactor
- 66: Separation means
- 68: Inlet line for fatty acid
- 70: Inlet line for isopropanol
- 72: Outlet line for pre-reaction mixture
- 74: Outlet line for (final) reaction mixture
- 76: Overhead outlet line the second reactor
- 78: Line
- 80: Water removal unit
- 82: Outlet line for water
- 84: Outlet line for isopropanol
- 86: Recycle line
- 88: Overhead outlet line of separation means
- 90: Bottom line of separation means
- 92: Recycle line

## Claims

1. A method for purifying a fatty acid isopropyl ester comprising the following steps:
a) providing a crude fatty acid isopropyl ester composition containing a fatty acid isopropyl ester to be purified, at least one fatty acid and optionally at least one fatty acid ester being different from the fatty acid isopropyl ester to be purified, and
b) subjecting the crude fatty acid isopropyl ester composition provided in step a) to a distillation in a divided-wall column so as to produce an overhead stream, a bottom stream and a side stream, wherein the side stream is the purified fatty acid isopropyl ester composition.

2. The method in accordance with claim 1, wherein the crude fatty acid isopropyl ester composition provided in step a) contains as fatty acid isopropyl ester to be purified isopropyllaurate, isopropylmyristate, isopropylpalmitate, isopropylstearate or an arbitrary mixture of two or more of the aforementioned fatty acid isopropyl esters.

3. The method in accordance with claim 1 or 2, wherein the crude fatty acid isopropyl ester composition provided in step a) contains 20 to 80% by weight of the fatty acid isopropyl ester to be purified, 30 to 80% by weight of one or more fatty acids, 0.2 to 2.0% by weight of one or more fatty acid isopropyl esters being different from the fatty acid isopropyl ester to be purified, 0.01 to 0.2% by weight of isopropanol and up to 0.5% by weight of water, based on the total weight of the crude fatty acid isopropyl ester composition.

4. The method in accordance with any of the preceding claims, wherein the divided-wall column used in step b) comprises one dividing wall, which extends at least essentially vertically downwards, wherein essentially vertically downwards means that the angle between the dividing wall and the length axis of the divided-wall column is at most 20°, preferably at most 10°, more preferably at most 5° and most preferably 0°, wherein the dividing wall extends, seen along the length axis of the divided-wall column, from 10 to 90%, preferably from 20 to 80% and more preferably from 25 to 75% of the height of the divided-wall column.

5. The method in accordance with any of the preceding claims, wherein the divided-wall column used in step b) is a middle divided-wall column comprising:
- an undivided top section for concentrating low-boiling components comprising an outlet for the overhead stream of the low-boiling components, wherein the low-boiling components have a lower boiling point than the fatty acid isopropyl ester to be purified,
- an undivided bottom section for concentrating high-boiling components comprising an outlet for the bottom stream of the high-boiling components, wherein the high-boiling components have a higher boiling point than the fatty acid isopropyl ester to be purified, and
- a central section arranged between the top section and the bottom section having at least one dividing wall for dividing the central section into at least one feed sub-section and at least one product withdrawal sub-section, the feed sub-section having an inlet for the feed of the crude fatty acid isopropyl ester composition and the product withdrawal sub-section having a side draw outlet for the purified fatty acid isopropyl ester composition.

6. The method in accordance with claim 5, wherein the side draw outlet for the purified fatty acid isopropyl ester composition is arranged in the central section of the middle divided-wall column above the inlet for the feed of the crude fatty acid isopropyl ester composition.

7. The method in accordance with claim 5 or 6, wherein each of the feed sub-section and of the product withdrawal sub-section of the central section of the divided-wall column comprises at least two packing beds, more preferably at least three packing beds and most preferably four packing beds, wherein adjacent packing beds are arranged on top of each other, and wherein each of the packing beds preferably comprises one or more structured packing elements or a random packing.

8. The method in accordance with claim 7, wherein the inlet for the feed of the crude fatty acid isopropyl ester composition is located between two adjacent packing beds of the feed sub-section and the outlet for the purified fatty acid isopropyl ester composition is located between two adjacent packing beds of the product withdrawal sub-section, wherein the side draw outlet for the purified fatty acid isopropyl ester composition is arranged in the central sub-section of the middle divided-wall column above the inlet for the feed of the crude fatty acid isopropyl ester composition.

9. The method in accordance with any one of the preceding claims, wherein the reflux ratio is 200 to 1,000, preferably 300 to 900 and more preferably 500 to 800, and/or wherein the ratio of the reflux rate divided by the side stream rate is 4 to 10, preferably 5 to 8 and more preferably 6 to 7.

10. The method in accordance with any one of the preceding claims, wherein no further distillation is performed in addition to that performed in the divided-wall column.

11. The method in accordance with any one of the preceding claims, wherein the purified fatty acid isopropyl ester composition obtained in step b) as side stream comprises at least 99.00% by weight, preferably at least 99,30%, more preferably at least 99,50%, even more preferably at least 99,60% and most preferably at least 99,70% of the target fatty acid isopropyl ester, wherein the purified fatty acid isopropyl ester composition obtained in step b) as side stream comprises at most 0.070% by weight, preferably at most 0.060% by weight, more preferably at most 0.050% by weight, even more preferably at most 0.040% by weight and most more preferably at most 0.030% by weight of impurities comprising free fatty acid and other fatty acid isopropyl esters than the fatty acid isopropyl ester to be purified.

12. A method for preparing a pure fatty acid isopropyl ester comprising the following steps:
i) providing a starting mixture containing a fatty acid and isopropanol,
ii) reacting the starting mixture preferably in the presence of a catalyst so as to obtain a reaction mixture containing the fatty acid isopropyl ester, unreacted fatty acid, unreacted isopropanol and optionally water, by-products and other impurities,
iii) optionally, removing at least a part of the isopropyl from the reaction mixture by vaporization so as to obtain a modified reaction mixture, and
iv) performing a method for purifying the fatty acid isopropyl ester in accordance with any of the preceding claims, wherein the crude fatty acid isopropyl ester composition provided in step a) is the reaction mixture obtained in step ii) or the modified reaction mixture obtained in step iii).

13. A plant for preparing a pure fatty acid isopropyl ester comprising:
i) at least one reactor one or more inlets for fatty acid and isopropanol or a mixture of fatty acid and isopropanol, and an outlet for a reaction mixture containing the fatty acid isopropyl ester, unreacted fatty acid, unreacted isopropanol and optionally water, by-products and other impurities,
ii) optionally, a separation means for separating at least a part of isopropanol from the reaction mixture so as to obtain a modified reaction mixture, wherein the separation means comprises an inlet being connected with the outlet for the reaction mixture of the reactor, an outlet for isopropanol and an outlet for the modified reaction mixture, and
iii) a divided-wall column comprising an inlet being connected with the outlet for the reaction mixture of the reactor and/or with the outlet for the modified reaction mixture of the separation means.

14. The plant in accordance with claim 13, wherein the reactor a) is a distillation column comprising as bottom outlet the outlet for the reaction mixture and an overhead outlet, wherein the overhead outlet is connected via a recycle line with a water removal unit, wherein the water removal unit comprises an outlet for water and an outlet for isopropanol, wherein the outlet for isopropanol is connected via a recycle line with the inlet for isopropanol of the reactor a).

15. The plant in accordance with claim 13 or 14, wherein the separation means b) is present, wherein the outlet for isopropanol of the separation means b) is connected via a recycle line with the inlet for isopropanol of the reactor a).
